# EUROPEAN PATENT APPLICATION

(11) **EP 4 617 356 A1**
(43) Date of publication of application: **17.09.2025**
(21) Application number: 24163597.8
(22) Date of filing: 14.03.2024
(51) Int. Cl.: C12M 1/36, C12M 1/26, G01N 1/02

(54) **METHOD OF CONTROLLING A BIOPROCESS ARRANGEMENT TO PROVIDE MEASUREMENT DATA TO A BIOPROCESS CONTROL SYSTEM**

(71) Applicant: Sartorius Stedim Biotech GmbH, 37079 Göttingen (DE)
(72) Inventor: Scholz, Jochen, 37077 Göttingen (DE); Grimm, Christian, 37308 Heilbad Heiligenstadt (DE)
(74) Representative: Gottschald Patentanwälte Partnerschaft mbB

(57) **Abstract**

The invention relates to a method of controlling a bioprocess arrangement (1) to provide measurement data to a bioprocess control system (3), wherein the bioprocess arrangement (1) comprises a biocontainer arrangement (2), a sampling arrangement (4), a routing arrangement (6) and an analysis arrangement (7). It is proposed that the bioprocess control system (3) sends a measurement request to the control layer (9), that the measurement request contains information about a requested measurement, that the measurement request is independent of routing and analysis behind the sampling arrangement (4), that the sampling arrangement (4) draws the sample and provides the sample to the routing arrangement (6), that the control layer (9) coordinates the provision of the sample to the analysis arrangement (7) via the routing arrangement (6), that the analysis arrangement (7) sends measurement data to the control layer (9), and, that the control layer (9) sends the measurement data to the bioprocess control system (3).

## Description

The present invention relates to a method of controlling a bioprocess arrangement to provide measurement data to a bioprocess control system according to the general part of claim 1 and to a control system implementing a control layer according to claim 15.

The control and analysis of a bioprocess often involve taking samples of a cell broth or other fluids used in the bioprocess. Sampling and analyzing a sample are cost- and often labor-intensive process steps compared to online measurements of e.g. temperature or pH. However, a number of relevant measurements cannot be taken online and need dedicated analysis devices.

WO 2021/176106 A1 discloses a system for inline sampling to determine critical process parameters and critical quality attributes quickly enough to be able to influence the bioprocess while still running and possibly prevent the bioprocess from going out-of-spec. Such sampling may also be called intime as it is not in real time, but still close enough. The system is adapted to periodically sample a bioreactor and provide measurements from the sample. Problematic with such a system is that each sampling removes a part of the bioreactors content. This may be acceptable, but is still costly, for large volumes but can become very problematic for small volumes, for example for systems like the Sartorius Ambr. Further, the complexity of an automatic sampling system in setup and use is very high due to the interaction of many components with very different functions and possibly different manufacturers.

It is a challenge to provide a flexible system architecture for the analysis of samples which is adapted to be used with different analysis devices and to provide the possibility of on-demand sampling, thereby reducing the number of samples compared to regular sampling.

The invention is based on the problem of providing such a system architecture.

The above-noted object is solved by the features of the characterizing part of claim 1.

The invention relates to a method of controlling a bioprocess arrangement to provide measurement data to a bioprocess control system, wherein the bioprocess arrangement comprises a biocontainer arrangement, a sampling arrangement, a routing arrangement and an analysis arrangement, wherein the bioprocess arrangement comprises a control layer, wherein the biocontainer arrangement comprises the bioprocess control system and at least one biocontainer, in particular bioreactor, controlled by the bioprocess control system, wherein the sampling arrangement draws samples from the biocontainer, wherein the analysis arrangement comprises at least one analysis device for analyzing the samples and generating the measurement data, and, wherein the routing arrangement comprises at least one fluid connection for transporting the samples between the sampling arrangement and the analysis arrangement.

A biocontainer is any vessel, tube, or the like or combination thereof that contains a fluid used in the bioprocess. Preferably the biocontainer is a bioreactor or a combination of a bioreactor and tubes. Anyhow, the biocontainer allows sampling of the fluid, directly or indirectly via another biocontainer.

The main realization of the present invention is that a virtual analyzer, being a system that acts as a sensor or analysis system, may be provided to simplify the overall control of a bioprocess arrangement. The virtual analyzer can be connected to a bioprocess control system as if it were a real analyzer or sensor. The bioprocess control system can then request a measurement from the virtual analyzer and provide a sample to be analyzed to the virtual analyzer. On a physical level, the virtual analyzer may comprise routing (e.g. tubes and tube connections) and sample preparation and analysis devices for one or more bioprocess control systems. The virtual analyzer comprises a control layer which implements the physical control of the components of the virtual analyzer and preferably provides a single point-of-contact on a communications level. This allows flexibly adding and removing components from the virtual analyzer and connecting it to several bioprocess control systems, without increasing the complexity of the bioprocess control system. At the same time, it is also not necessary to integrate the bioprocess control into the control layer. By reducing the number of samples and allowing on-demand sampling, the number of analysis devices per bioprocess arrangement can be reduced.

In detail, it is proposed that the bioprocess control system sends a measurement request to the control layer, that the measurement request contains information about a requested measurement, that the measurement request is independent of routing and analysis behind the sampling arrangement, that the sampling arrangement draws the sample and provides the sample to the routing arrangement, that the control layer coordinates the routing arrangement and the analysis arrangement to provide the sample to the analysis arrangement via the routing arrangement, that the analysis arrangement analyzes the sample and generates the measurement data, that the analysis arrangement sends the measurement data to the control layer, and, that the control layer sends the measurement data to the bioprocess control system.

In a preferred embodiment according to claim 2, physical and virtual control of the sample are handed over at a physical sampling interface. The bioprocess control system then only needs to know where to provide the sample to and possibly provide some information and the virtual analyzer receives the sample. The means by which the virtual analyzer routes and analyses the sample are not relevant for the bioprocess control system and can be configured, physically and virtually at the control layer, as needed.

The sampling arrangement fits logically into the virtual analyzer but several biocontainer arrangements, e.g. the Ambr, already provide for sampling. Claim 3 therefore relates to both alternatives and proposes using one interface for several biocontainers. Especially in the case of several jointly controlled bioreactors, a joint sampling arrangement may be very efficient.

The bioprocessing arrangement may comprise a storage arrangement and/or a sample preparation arrangement which advantageously can be coordinated by the control layer and may therefore not be visible for the bioprocess control system (claims 4 and 5).

In an embodiment according to claim 6 at least some of the arrangements controlled by the control layer do not communicate with each other, further increasing the modularity of the bioprocess arrangement. The control layer may have drivers for different components of different arrangements and may be the single point of contact for some or even all devices.

A routing arrangement may be the center of the physical connections, sending the sample or possible aliquots back and forth between the other arrangements. The modularity can be increased if no or few direct connections between arrangements are provided (claim 7).

In a preferred embodiment according to claim 8, the control layer handles several samples, in particular at the same time. The control layer may also implement a mechanism for prioritization of samples, possibly based on information in the measurement request.

During configuration of the communication between the bioprocess control system and the control layer, the control layer may identify as one or more virtual analyzers, for example by imitating a physical analyzer connected to the bioprocess control system according to an embodiment of claim 9.

The control layer may comprise a planning layer and a driver layer (claim 10). Such layers are independent in a way that for example a driver may be exchanged without affecting the planning layer if all abilities of the device or arrangement controlled by the driver stay the same.

In one embodiment according to claim 11, the control layer provides, upon request, a processing parameter for a measurement request or provides several possible processing parameters to choose from, if possible, for example by choosing higher or lower accuracy or higher or lower measurement effort or costs. The bioprocessing control system is thereby enabled to choose measurements depending on different constraints.

The control layer may further gather an audit trail by combining data from the different arrangements (claim 12).

The virtual analyzer or analyzers may be organized such that a single measurement request is performed by different analysis devices of the analysis arrangement orchestrated by the control layer (claim 13).

Claim 14 relates to possible reactions of the bioprocess control to the measurement data.

Another teaching according to claim 15, which is of equal importance, relates to a control system implementing the control layer adapted to be used in the proposed method.

All explanations given with regard to the proposed method are fully applicable.

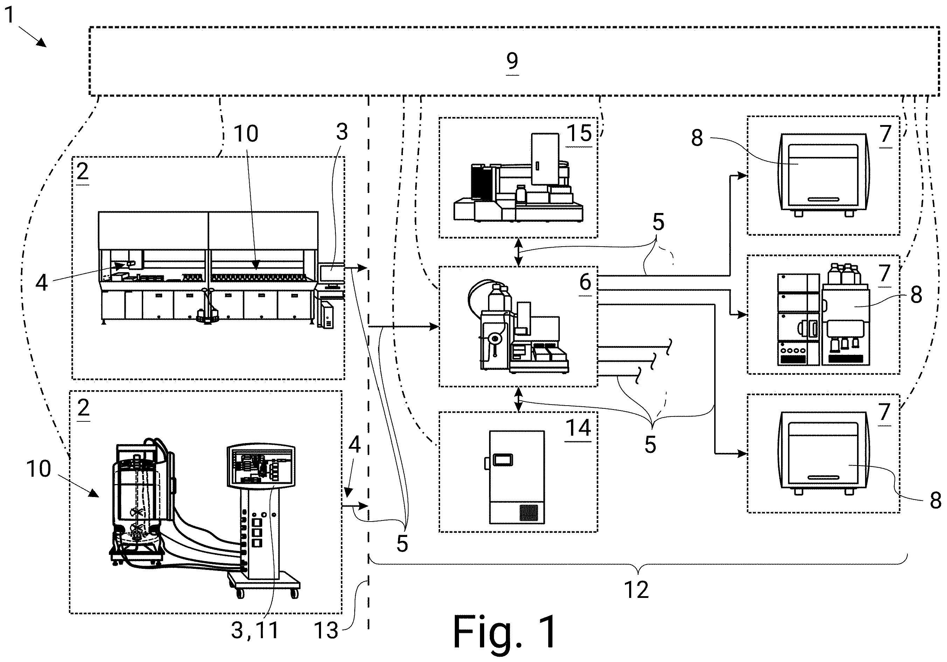

In the following, embodiments of the invention are explained with respect to the drawing. The drawing shows in
- Fig. 1,: a proposed bioprocess arrangement.

Fig. 1 shows a bioprocess arrangement 1 comprising two biocontainer arrangements 2, the top one representing a Sartorius Ambr 250 High Throughput, the bottom one any bioreactor.

Proposed is a method of controlling a bioprocess arrangement 1 to provide measurement data to a bioprocess control system 3. The bioprocess arrangement 1 comprises at least one biocontainer arrangement 2. Further shown and comprised by the bioprocess arrangement 1 are a sampling arrangement 4 (shown exemplarily as part of the Ambr and not shown as part of the bioreactor but conceptually part of the arrow indicating a fluid connection 5 leaving the bioreactor), a routing arrangement 6 (in the middle) and an analysis arrangement 7, here comprising exemplarily three analysis devices 8. Alternative biocontainer arrangements 2 may comprise mixing containers, surge tanks, filtration arrangements, preparative chromatography arrangements and the like.

The bioprocess arrangement 1 comprises a control layer 9 shown at the top of Fig. 1. The control layer 9 is a software running on a laboratory information management system or any other computer system. The biocontainer arrangement 2 comprises the bioprocess control system 3 and at least one biocontainer 10, in particular bioreactor, controlled by the bioprocess control system 3. The shown Ambr is connected to a bioprocess control system 3 shown next to it and/or may comprise a bioprocess control system 3. The bioreactor comprises a digital control unit 11 shown next to the bioreactor as a bioprocess control system 3. These are merely exemplary. Here and preferably and as indicated by the Ambr, the biocontainer arrangement 2 may comprise several bioreactors, possibly more than ten or even more than 20. In particular for cases with several bioreactors or several biocontainers 10, possibly connected into a continuous process line, e.g. by having at least one bioreactor and at least one downstream processing arrangement as part of the bioprocess arrangement 1, the high complexity for sampling from different, for example at least four, biocontainers 10, can be handled well by the proposed solution.

The sampling arrangement 4 draws samples from the biocontainer 10, either directly or by drawing the sample from a tube connected to or forming part of the biocontainer 10 or the like.

The analysis arrangement 7 comprises at least one analysis device 8 for analyzing the samples and generating the measurement data. The routing arrangement 6 comprises at least one fluid connection 5 for transporting the samples between the sampling arrangement 4 and the analysis arrangement 7.

Proposed is that the bioprocess control system 3 sends a measurement request to the control layer 9, that the measurement request contains information about a requested measurement and that the measurement request is independent of routing and analysis behind the sampling arrangement 4. Here and preferably, the control layer 9 is the single point of contact for measurement requests related to the analysis devices 8 of the analysis arrangement 7. As will be explained, the control layer 9 may have presented to the bioprocess control system 3 as being or comprising one or more virtual analyzers 12, possibly even without disclosing that the analyzer is virtual. The bioprocess control system 3 may address the control layer 9 as it would address any sample-based analyzer to receive measurement data. The bioprocess control system 3 is not involved in controlling the analysis devices 8.

The sampling arrangement 4 draws the sample and provides the sample to the routing arrangement 6. The control layer 9 coordinates the routing arrangement 6 and the analysis arrangement 7 to provide the sample to the analysis arrangement 7 via the routing arrangement 6, in particular directly from the sampling arrangement 4 or by controlling the sampling arrangement 4. The analysis arrangement 7 analyzes the sample and generates the measurement data. The analysis arrangement 7 sends the measurement data to the control layer 9 and the control layer 9 sends the measurement data to the bioprocess control system 3. The measurement data may relate to critical quality attributes and/or critical process parameters.

Here and preferably the routing arrangement 6, the analysis arrangement 7 and the biocontainer arrangement 2 are all separate entities with defined physical and virtual connections including a handover of the sample and of communications between the separate entities. The measurement request here and preferably contains a desired measurement and parameters of the measurement e.g. an accuracy or precision.

Here and preferably, the control layer 9 assigns a sample ID to each sample and/or aliquot or receives a sample ID from the bioprocess control system 3.

It may be the case that the data from the analysis arrangement 7 is sent to the control layer 9 via the routing arrangement 6. In general, any communication may be forwarded through one or more arrangements, preferably without changes.

Here and preferably the control layer 9 acts as one or more virtual analyzers 12 for several bioprocess control systems 3 using the same analysis arrangement 7 and routing arrangement 6.

It is preferably the case that the sampling arrangement 4 comprises a sampling interface 13 comprising a fluid connection 5 fluidically connected to the routing arrangement 6. The sampling interface 13 is depicted only schematically as a dotted line in Fig. 1. It should be understood that the sampling interface 13 of the shown Ambr may be a fluid port connected to a sampling cup or the like of the Ambr. As is known, an Ambr comprises a sampling robot as the sampling arrangement 4.

The sampling arrangement 4 provides the sample to the sampling interface 13 and control of the sample is handed over from the sampling arrangement 4 to the routing arrangement 6. For the Ambr case, the sampling robot puts the sample in the sampling cup or the like and from there the sample moves through the fluid port into for example a tube controlled by the routing arrangement 6. Preferably, the measurement request contains a handover description containing physical parameters of the handover and the control layer 9 coordinates the handover. The physical parameters may comprise a time of the handover and/or possibly a port for the handover if there are multiple and/or a size of the sample. Here and preferably, with the handover the knowledge of the bioprocess control system 3 about the whereabouts and processing of the sample ends.

It may be the case, that the control layer 9 coordinates the sampling arrangement 4, or, that the sampling arrangement 4 is controlled by the bioprocess control system 3. As the Ambr example clarifies, the sampling arrangement 4 may be deeply intertwined with the biocontainer arrangement 2 and therefore controlled by the bioprocess control system 3.

Here and preferably, the biocontainer arrangement 2 comprises a multitude of bioreactors. The sampling arrangement 4 may comprise a sampling probe for the multitude of bioreactors. Preferably, samples of different bioreactors are sent through the same fluid connection 5 of the sampling interface 13 to the routing arrangement 6. The sampling arrangement 4 and/or the routing arrangement 6 may comprise a pump system for aseptic transfer of the sample. The sample provided may be a processed or partially processed sample, e.g. clarification and/or purification may be done before handover to the routing arrangement 6 or afterwards.

According to one embodiment it is proposed, that the bioprocess arrangement 1 comprises a storage arrangement 14 for storing the samples. Additionally or alternatively, the bioprocess arrangement 1 may comprise a sample preparation arrangement 15 for preparing the samples. Both are shown in Fig. 1. Preferably, the control layer 9 coordinates the storage arrangement 14 and/or the sample preparation arrangement 15.

Here and preferably, the storage arrangement 14 stores one or more samples in a controlled environment, in particular in a temperature controlled environment. The samples may be stored at 4° C to 10° C or -40° C to 0° C. It is therefore depicted as a refrigerator but may in particular additionally or alternatively comprise a storage facility for storage of the samples at a temperature above 20° C or above 30° C.

The sample preparation arrangement 15 here and preferably performs one or more of: clarification of the sample from cells, purification of the sample, dilution of the sample, digestion of the sample, chemical treatment of the sample, aliquoting of the sample, centrifuging of the sample. Additionally or alternatively, the routing arrangement 6 may also be equipped for aliquoting the sample and/or diluting the (aliquoted) samples and do so on demand.

Here and preferably, two or more of the sampling arrangement 4, the analysis arrangement 7, the storage arrangement 14, the sample preparation arrangement 15 and the routing arrangement 6 do no communicate which each other and preferably communicate with the control layer 9 independent of each other. Fig. 1 shows dotted lines as data connections between the control layer 9 and the different arrangements.

The control layer 9 may send requests to one or more of the arrangements containing information about a sample to be received and/or processed and/or handed over and may coordinate a handover of control over the physical sample between arrangements.

It is preferably the case that the routing arrangement 6 is fluidically connected to and routes samples between one or more of the sampling arrangement 4, the analysis arrangement 7, the storage arrangement 14 and the sample preparation arrangement 15. Preferably and as shown in Fig. 1, one or more of, here all of, the sampling arrangement 4, the analysis arrangement 7, the storage arrangement 14 and the sample preparation arrangement 15 are fluidically connected only through the routing arrangement 6.

According to one embodiment it is proposed, that the control layer 9 plans the routing and optionally sampling and/or storage of several samples, in particular several samples being processed by the bioprocess arrangement 1 at the same time. These samples may have been taken from the same biocontainer 10 or several biocontainers 10. Some samples may be stored for extended periods of time while other samples may be processed quickly.

Preferably, the control layer 9 prioritizes some samples over others and plans the routing and optionally storage accordingly. For that, the measurement request may contain a target processing time for the measurement and/or a relevance of the measurement and the control layer 9 then prioritizes the samples based on the target processing time and/or the relevance.

The measurement request may contain information about, in particular including at least one boundary for, accuracy and/or processing time of an analysis. The control layer 9 may then choose an analysis device 8 and optionally a routing and/or sample preparation steps and/or an aliquoting based on this information, in particular to meet the boundary. Measurement requests with the same content may therefore be processed by different analysis devices 8 with different accuracies based on the availability of the analysis devices 8. The measurement request may further contain information about the sample origin, in particular which bioreactor in the Ambr system the sample was taken from at which time in the bioprocess. This information may be transmitted together with the sample and included in a measurement report or the measurement data in general.

During a configuration routine of the bioprocess arrangement 1 the control layer 9 may identify to the bioprocess control system 3 as one or more virtual analyzers 12 and the bioprocess control system 3 may later send the measurement request to one of the virtual analyzers 12 independent of the physical components of the virtual analyzer 12.

It is possible that the control layer 9 runs on one or more computer systems independent of the bioprocess control system 3. The control layer 9 may comprise a planning layer which plans the execution of the measurement requests and a driver layer which receives requests from the planning layer and executes the requests by communicating with the physical components of the bioprocess arrangement 1. The layers may function independently of each other and communicate through standardized data protocols. Preferably, the driver layer comprises drivers for the physical components. The control layer 9 may be coupled to the bioprocess control system 3 via a bus and/or at a data connection slot for an analyzer.

According to one embodiment it is proposed, that the bioprocess control system 3 requests a processing parameter, in particular processing time, for a measurement request from the control layer 9 and that the control layer 9 provides the processing parameter. It may then be the case that the control layer 9 provides several possible processing parameters for different possible measurements with different further parameters, in particular different accuracies of the measurement and that the bioprocess control system 3 chooses one of the several processing parameters. The provided processing parameters may depend on the availability of the analysis arrangement 7.

It is further preferred that the control layer 9 gathers an audit trail report for a sample from the routing arrangement 6, the analysis arrangement 7 and optionally the storage arrangement 14 and/or the sample preparation arrangement 15. The control layer 9 may provide the audit trail report to the bioprocess control system 3. The audit trail may include data from the measurement request, in particular a sample ID and/or sample origin.

According to one embodiment it is proposed, that the measurement request contains several requested measurements which are performed by different analysis devices 8 of the analysis arrangement 7, that the control layer 9 determines and organizes a necessary taking and routing of one or more samples to fulfill the measurement request and/or a necessary aliquoting and routing for the sample to fulfill the measurement request, preferably from a single sample.

Here and preferably the control layer 9 executes an optimization routine to determine the taking and/or aliquoting and routing of the sample and a time schedule for the analysis devices 8. In the optimization routine the control layer 9 may take into account the type and number of the analysis devices 8 and/or constraints for different samples and/or states of the analysis devices 8 and/or a possible re-routing in case of an error, preferably to achieve a minimized overall processing time and/or a certain workload for a certain analyzer and/or for resolving conflicts.

According to one embodiment it is proposed, that the bioprocess control system 3 controls the bioprocess based on the measurement data and/or performs a recalibration of a sensor used in the bioprocess based on the measurement data and/or performs a quality release decision. The sensor being recalibrated may be an inline sensor like on of a pH, glucose or lactate sensor, a spectrometer, preferably Raman spectrometer, a UV-Vis absorption spectrometer or photometer, or a NIR or MIR spectrometer which is recalibrated based on external reference measurements.

Another teaching which is of equal importance relates to a control system implementing the control layer 9 adapted to be used in the proposed method.

## Claims

1. Method of controlling a bioprocess arrangement (1) to provide measurement data to a bioprocess control system (3), wherein the bioprocess arrangement (1) comprises a biocontainer arrangement (2), a sampling arrangement (4), a routing arrangement (6) and an analysis arrangement (7), wherein the bioprocess arrangement (1) comprises a control layer (9), wherein the biocontainer arrangement (2) comprises the bioprocess control system (3) and at least one biocontainer (10), in particular bioreactor, controlled by the bioprocess control system (3), wherein the sampling arrangement (4) draws samples from the biocontainer (10), wherein the analysis arrangement (7) comprises at least one analysis device (8) for analyzing the samples and generating the measurement data, and, wherein the routing arrangement (6) comprises at least one fluid connection (5) for transporting the samples between the sampling arrangement (4) and the analysis arrangement (7),
**characterized in**
**that** the bioprocess control system (3) sends a measurement request to the control layer (9), that the measurement request contains information about a requested measurement, that the measurement request is independent of routing and analysis behind the sampling arrangement (4), that the sampling arrangement (4) draws the sample and provides the sample to the routing arrangement (6), that the control layer (9) coordinates the routing arrangement (6) and the analysis arrangement (7) to provide the sample to the analysis arrangement (7) via the routing arrangement (6), that the analysis arrangement (7) analyzes the sample and generates the measurement data, that the analysis arrangement (7) sends the measurement data to the control layer (9), and, that the control layer (9) sends the measurement data to the bioprocess control system (3).

2. Method according to claim 1, **characterized in that** the sampling arrangement (4) comprises a sampling interface (13) comprising a fluid connection (5) fluidically connected to the routing arrangement (6), that the sampling arrangement (4) provides the sample to the sampling interface (13) and control of the sample is handed over from the sampling arrangement (4) to the routing arrangement (6), preferably, that the measurement request contains a handover description containing physical parameters of the handover and the control layer (9) coordinates the handover.

3. Method according to claim 1 or 2, **characterized in that** the control layer (9) coordinates the sampling arrangement (4), or, that the sampling arrangement (4) is controlled by the bioprocess control system (3), and/or, that the biocontainer arrangement (2) comprises a multitude of bioreactors, that the sampling arrangement (4) comprises a sampling probe for the multitude of bioreactors, preferably, that samples of different bioreactors are sent through the same fluid connection (5) of the sampling interface (13) to the routing arrangement (6).

4. Method according to one of the preceding claims, **characterized in that** the bioprocess arrangement (1) comprises a storage arrangement (14) for storing the samples, and/or, that the bioprocess arrangement (1) comprises a sample preparation arrangement (15) for preparing the samples, preferably, that the control layer (9) coordinates the storage arrangement (14) and/or the sample preparation arrangement (15).

5. Method according to claim 4, **characterized in that** the storage arrangement (14) stores one or more samples in a controlled environment, in particular in a temperature controlled environment, and/or, that the sample preparation arrangement (15) performs one or more of: clarification of the sample from cells, purification of the sample, dilution of the sample, digestion of the sample, chemical treatment of the sample, aliquoting of the sample, centrifuging of the sample.

6. Method according to one of the preceding claims, **characterized in that** two or more of the sampling arrangement (4), the analysis arrangement (7), the storage arrangement (14), the sample preparation arrangement (15) and the routing arrangement (6) do no communicate which each other and preferably communicate with the control layer (9) independent of each other.

7. Method according to one of the preceding claims, **characterized in that** the routing arrangement (6) is fluidically connected to and routes samples between one or more of the sampling arrangement (4), the analysis arrangement (7), the storage arrangement (14) and the sample preparation arrangement (15), preferably, that one or more of the sampling arrangement (4), the analysis arrangement (7), the storage arrangement (14) and the sample preparation arrangement (15) are fluidically connected only through the routing arrangement (6).

8. Method according to one of the preceding claims, **characterized in that** the control layer (9) plans the routing and optionally sampling and/or storage of several samples, in particular several samples being processed by the bioprocess arrangement (1) at the same time, preferably, that the control layer (9) prioritizes some samples over others and plans the routing and optionally storage accordingly, more preferably, that the measurement request contains a target processing time for the measurement and/or a relevance of the measurement and the control layer (9) prioritizes the samples based on the target processing time and/or the relevance.

9. Method according to one of the preceding claims, **characterized in that** during a configuration routine of the bioprocess arrangement (1) the control layer (9) identifies to the bioprocess control system (3) as one or more virtual analyzers (12) and that the bioprocess control system (3) sends the measurement request to one of the virtual analyzers (12) independent of the physical components of the virtual analyzer (12).

10. Method according to one of the preceding claims, **characterized in that** the control layer (9) runs on one or more computer systems independent of the bioprocess control system (3), that the control layer (9) comprises a planning layer which plans the execution of the measurement requests and a driver layer which receives requests from the planning layer and executes the requests by communicating with the physical components of the bioprocess arrangement (1), preferably, that the driver layer comprises drivers for the physical components.

11. Method according to one of the preceding claims, **characterized in that** the bioprocess control system (3) requests a processing parameter, in particular processing time, for a measurement request from the control layer (9), that the control layer (9) provides the processing parameter, preferably, that the control layer (9) provides several possible processing parameters for different possible measurements with different further parameters, in particular different accuracies of the measurement, that the bioprocess control system (3) chooses one of the several processing times.

12. Method according to one of the preceding claims, **characterized in that** the control layer (9) gathers an audit trail report for a sample from the routing arrangement (6), the analysis arrangement (7) and optionally the storage arrangement (14) and/or the sample preparation arrangement (15), preferably, that the control layer (9) provides the audit trail report to the bioprocess control system (3).

13. Method according to one of the preceding claims, **characterized in that** the measurement request contains several requested measurements which are performed by different analysis devices (8) of the analysis arrangement (7), that the control layer (9) determines and organizes a necessary taking and routing of one or more samples to fulfill the measurement request and/or a necessary aliquoting and routing for the sample to fulfill the measurement request, preferably from a single sample.

14. Method according to one of the preceding claims, **characterized in that** the bioprocess control system (3) controls the bioprocess based on the measurement data and/or performs a recalibration of a sensor used in the bioprocess based on the measurement data and/or performs a quality release decision.

15. Control system implementing the control layer (9) adapted to be used in the method according to one of claims 1 to 14.
